# EUROPEAN PATENT APPLICATION

(11) **EP 4 711 783 A1**
(43) Date of publication of application: **18.03.2026**
(21) Application number: 25200526.9
(22) Date of filing: 05.09.2025
(51) Int. Cl.: G01R 31/36, G01R 31/367

(54) **METHOD AND SYSTEM FOR PREDICTING A PERFORMANCE OF A SECONDARY BATTERY**

(30) Priority: 13.09.2024 KR 20240126119
(71) Applicant: SAMSUNG SDI CO., LTD., Yongin-si, Gyeonggi-do 17084 (KR)
(72) Inventor: KIM, Hyunkyu, 17084 Yongin-si (KR); YI, Sangkoan, 17084 Yongin-si (KR); KO, Younghoon, 17084 Yongin-si (KR); JANG, Junho, 17084 Yongin-si (KR); YEON, Dong Hee, 17084 Yongin-Si (KR)
(74) Representative: Maiwald GmbH

(57) **Abstract**

A method of predicting an electrical performance of a secondary battery. The method includes receiving design conditions of the secondary battery, receiving experiment data of the secondary battery, obtaining model parameters based on the experiment data and an electrochemical model, generating an electrochemical model library including the model parameters, and predicting the electrical performance of the secondary battery, having the design conditions, based on the electrochemical model library. The design conditions of the secondary battery include at least one of an electrode condition or an active material condition.

## Description

### BACKGROUND

### Field

The present disclosure relates to a method of predicting a performance of a secondary battery and a system for predicting a performance of a secondary battery using the same, and more particularly, to a method of predicting an electrical performance of a secondary battery according to design conditions based on an electrochemical model library and a system for predicting an electrical performance of a secondary battery using the same.

### Description of the Related Art

Unlike primary batteries that are not designed to be (re)charged, secondary (or rechargeable) batteries are batteries that are designed to be discharged and recharged. Low-capacity secondary batteries are used in portable, small electronic devices, such as smart phones, feature phones, notebook computers, digital cameras, and camcorders, while large-capacity secondary batteries are widely used as power sources for driving motors in hybrid vehicles and electric vehicles and for storing power (e.g., home and/or utility scale power storage). A secondary battery generally includes an electrode assembly composed of a positive electrode and a negative electrode, a case accommodating the same, and electrode terminals connected to the electrode assembly.

In the initial design of a secondary battery, predicting the performance of the secondary battery may be important. There are various models for predicting the performance of secondary batteries, such as, for example, models using morphology or equivalent circuit models. To accurately simulate the electrochemical performance of a secondary battery, a process of fitting model parameters with design conditions may be important. However, it may be not efficient to fit model parameters with design conditions for experiments when the design conditions change time after time.

The above information disclosed in this Background section is for enhancement of understanding of the background of the present disclosure, and therefore, it may contain information that does not constitute related (or prior) art.

### SUMMARY

To solve the above problem, the present disclosure provides a method of predicting a performance of a secondary battery and a system for predicting a performance of a secondary battery.

These and other aspects and features of the present disclosure will be described in or will be apparent from the following description of embodiments of the present disclosure.

Some embodiments of the present disclosure include a method of predicting an electrical performance of a secondary battery. The method includes receiving design conditions of the secondary battery, receiving experiment data of the secondary battery, obtaining model parameters based on the experiment data and an electrochemical model, generating an electrochemical model library having the model parameters, and predicting the electrical performance of the secondary battery, having the design conditions, based on the electrochemical model library. The design conditions of the secondary battery include at least one of an electrode condition or an active material condition.

According to an embodiment of the present disclosure, the electrical performance of the secondary battery includes at least one of a charge capacity, a discharge capacity, or a C-rate characteristic of the secondary battery.

According to an embodiment of the present disclosure, the method further includes generating prediction data by applying the experiment data to the electrochemical model, optimizing the model parameters by comparing the experiment data and the prediction data, and updating the electrochemical model library with the optimized model parameters.

According to an embodiment of the present disclosure, the method further includes generating input data of the electrochemical model based on the experiment data, obtaining the prediction data by applying the input data to the electrochemical model, and generating new input data through an optimization algorithm in response to determining that an error value between the experiment data and the prediction data is greater than or equal to a predetermined threshold value.

According to an embodiment of the present disclosure, generating new input data through the optimization algorithm includes generating new input data using at least one of a particle swarm optimization algorithm, a genetic algorithm, or a Bayesian algorithm.

According to an embodiment of the present disclosure, obtaining the model parameters includes obtaining a first model parameter associated with electrolyte properties, obtaining a second model parameter associated with active material properties, and obtaining a third model parameter associated with electrode plate properties.

According to an embodiment of the present disclosure, obtaining the model parameters includes obtaining a first model parameter related to electrolyte properties from first experiment data through an advanced electrolyte model simulation.

According to an embodiment of the present disclosure, obtaining the model parameters includes obtaining a second model parameter related to active material properties from second experiment data through a discrete element method simulation.

According to an embodiment of the present disclosure, obtaining the model parameters includes obtaining a third model parameter related to electrode plate properties from third experiment data through a Newman model.

According to an embodiment of the present disclosure, receiving design conditions of the second battery includes receiving the active material condition, the active material condition including a condition for a mixed material in which multiple ingredients having different properties are mixed.

Preferably, non-transitory computer-readable recording medium storing a computer program for executing a method according to an embodiment of the present disclosure may be provided.

Some embodiments of the present disclosure include a system for predicting an electrical performance of a secondary battery. The system includes a memory and at least one processor connected to the memory and configured to execute at least one computer-readable program stored in the memory. The at least one processor is configured to receive design conditions of the secondary battery, receive experiment data of the secondary battery, obtain model parameters based on the experiment data and an electrochemical model, generate an electrochemical model library comprising the model parameters, and predict the electrical performance of the secondary battery, having the design conditions, based on the electrochemical model library. The design conditions include at least one of an electrode condition or an active material condition.

According to an embodiment of the present disclosure, the electrical performance of the secondary battery comprises at least one of a charge capacity, a discharge capacity, or a C-rate characteristic of the secondary battery.

According to an embodiment of the present disclosure, the at least one processor is configured to generate prediction data by applying the experiment data to the electrochemical model, optimize the model parameters by comparing the experiment data and the prediction data, and update the electrochemical model library with the optimized model parameters.

Preferably, the at least one processor is configured to generate input data of the electrochemical model based on the experiment data, obtain the prediction data by applying the input data to the electrochemical model, and generate new input data through an optimization algorithm in response to a determination that an error value between the experiment data and the prediction data is greater than or equal to a predetermined threshold value.

Preferably, the optimization algorithm comprises at least one of a particle swarm optimization algorithm, a genetic algorithm, or a Bayesian algorithm.

Preferably, the at least one processor is configured to obtain a first model parameter related to electrolyte properties from first experiment data through an advanced electrolyte model simulation.

Preferably, the at least one processor is configured to obtain a second model parameter related to active material properties from second experiment data through a discrete element method simulation.

Preferably, the at least one processor is configured to obtain a third model parameter related to electrode plate properties from third experiment data through a Newman model.

Preferably, the active material condition includes a condition for a mixed material in which multiple ingredients having different properties are mixed.

Some embodiments of the present disclosure include a computer program comprising instructions which, when the program is executed by a computer, cause the computer to carry out the method as described herein.

Some embodiments of the present disclosure include a computer-readable medium comprising instructions which, when executed by a computer, cause the computer to carry out the method as described herein.

According to various embodiments of the present disclosure, the data predicted through the method of predicting secondary battery performance can exhibit a high level of accuracy when compared to data measured through experiments. Therefore, by predicting the electrical performance of a secondary battery under various conditions for the electrode plate and/or active material and managing the predictions using a library, it is possible to determine the electrical performance of the secondary battery without conducting a new experiment even if the design conditions are changed.

However, aspects and features of the present disclosure are not limited to those described above, and other aspects and features not mentioned will be clearly understood by a person skilled in the art from the detailed description, described below.

### BRIEF DESCRIPTION OF DRAWINGS

The following drawings attached to this specification illustrate embodiments of the present disclosure, and further describe aspects and features of the present disclosure together with the detailed description of the present disclosure. Thus, the present disclosure should not be construed as being limited to the drawings.
FIG. 1 is a schematic diagram of a system for predicting a performance of a secondary battery according to an embodiment of the present disclosure.
FIG. 2 is a block diagram of the internal structure of a processor of the system for predicting the performance of the secondary battery according to an embodiment of the present disclosure.
FIG. 3 is a diagram of an electrochemical model library of the system for predicting the performance of the secondary battery according to an embodiment of the present disclosure.
FIG. 4 is a diagram of a method of obtaining a first model parameter according to an embodiment of the present disclosure.
FIG. 5 is a diagram of a method of obtaining a second model parameter according to an embodiment of the present disclosure.
FIG. 6 is a diagram of a method of obtaining a third model parameter according to an embodiment of the present disclosure.
FIG. 7 shows graphs of data for predicting the performance of the secondary battery according to an embodiment of the present disclosure.
FIG. 8 is a method of predicting the performance of the secondary battery according to an embodiment of the present disclosure.
FIG. 9 is a process of updating the electrochemical model library according to an embodiment of the present disclosure.
FIG. 10 is a process of optimizing model parameters according to an embodiment of the present disclosure.

### DETAILED DESCRIPTION

Hereinafter, embodiments of the present disclosure will be described, in detail, with reference to the accompanying drawings. The terms or words used in this specification and claims should not be construed as being limited to the usual or dictionary meaning and should be interpreted as meanings and concepts that are consistent with the technical idea of the present disclosure based on the principle that the inventor can be his/her own lexicographer to appropriately define the concept of a term to explain his/her invention in the best way.

The embodiments described in this specification and the configurations shown in the drawings are only some of the embodiments of the present disclosure and do not represent all of the technical ideas, aspects, and features of the present disclosure. Accordingly, it should be understood that there may be various equivalents and modifications that can replace or modify the embodiments described herein at the time of filing this application.

It will be understood that when an element or layer is referred to as being "on," "connected to," or "coupled to" another element or layer, it may be directly on, connected, or coupled to the other element or layer or one or more intervening elements or layers may also be present. When an element or layer is referred to as being "directly on," "directly connected to," or "directly coupled to" another element or layer, there are no intervening elements or layers present. For example, when a first element is described as being "coupled" or "connected" to a second element, the first element may be directly coupled or connected to the second element or the first element may be indirectly coupled or connected to the second element via one or more intervening elements.

In the figures, dimensions of the various elements, layers, etc. may be exaggerated for clarity of illustration. The same reference numerals designate the same elements. As used herein, the term "and/or" includes any and all combinations of one or more of the associated listed items. Further, the use of "may" when describing embodiments of the present disclosure relates to "one or more embodiments of the present disclosure." Expressions, such as "at least one of" and "any one of," when preceding a list of elements, modify the entire list of elements and do not modify the individual elements of the list. When phrases such as "at least one of A, B and C, "at least one of A, B or C," "at least one selected from a group of A, B and C," or "at least one selected from among A, B and C" are used to designate a list of elements A, B and C, the phrase may refer to any and all suitable combinations or a subset of A, B and C, such as A, B, C, A and B, A and C, B and C, or A and B and C. As used herein, the terms "use," "using," and "used" may be considered synonymous with the terms "utilize," "utilizing," and "utilized," respectively. As used herein, the terms "substantially," "about," and similar terms are used as terms of approximation and not as terms of degree, and are intended to account for the inherent variations in measured or calculated values that would be recognized by those of ordinary skill in the art.

It will be understood that, although the terms first, second, third, etc. may be used herein to describe various elements, components, regions, layers, and/or sections, these elements, components, regions, layers, and/or sections should not be limited by these terms. These terms are used to distinguish one element, component, region, layer, or section from another element, component, region, layer, or section. Thus, a first element, component, region, layer, or section discussed below could be termed a second element, component, region, layer, or section without departing from the teachings of example embodiments.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" or "over" the other elements or features. Thus, the term "below" may encompass both an orientation of above and below. The device may be otherwise oriented (rotated 90 degrees or at other orientations), and the spatially relative descriptors used herein should be interpreted accordingly.

The terminology used herein is for the purpose of describing embodiments of the present disclosure and is not intended to be limiting of the present disclosure. As used herein, the singular forms "a" and "an" are intended to include the plural forms as well, unless the context clearly indicates otherwise. It will be further understood that the terms "includes," "including," "comprises," and/or "comprising," when used in this specification, specify the presence of stated features, integers, steps, operations, elements, and/or components but do not preclude the presence or addition of one or more other features, integers, steps, operations, elements, components, and/or groups thereof.

Also, any numerical range disclosed and/or recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein.

References to two compared elements, features, etc. as being "the same" may mean that they are "substantially the same". Thus, the phrase "substantially the same" may include a case having a deviation that is considered low in the art, for example, a deviation of 5% or less. In addition, when a certain parameter is referred to as being uniform in a given region, it may mean that it is uniform in terms of an average.

Throughout the specification, unless otherwise stated, each element may be singular or plural.

Arranging an arbitrary element "above (or below)" or "on (under)" another element may mean that the arbitrary element may be disposed in contact with the upper (or lower) surface of the element, and another element may also be interposed between the element and the arbitrary element disposed on (or under) the element.

In addition, it will be understood that when a component is referred to as being "linked," "coupled," or "connected" to another component, the elements may be directly "coupled," "linked" or "connected" to each other, or another component may be "interposed" between the components".

Throughout the specification, when "A and/or B" is stated, it means A, B or A and B, unless otherwise stated. That is, "and/or" includes any or all combinations of a plurality of items enumerated. When "C to D" is stated, it means C or more and D or less, unless otherwise specified.

In this specification, singular expressions may include plural expressions unless the context clearly specifies that they are singular. Also, plural expressions may include singular expressions unless the context clearly specifies that they are plural. Throughout the specification, when a part is said to include a certain component, this does not mean that it excludes other components, but rather that it may include other components, unless otherwise specifically stated.

In addition, the term 'module' or 'unit' used in the specification refers to a software or hardware component, and the 'module' or 'unit' performs specific roles. However, the 'module' or 'unit' is not limited to software or hardware. A 'module' or 'unit' may be configured to reside on an addressable storage medium and may be configured to drive one or more processors. Thus, as an example, a 'module' or 'unit' may include at least one of components such as software components, object-oriented software components, class components and task components, processes, functions, attributes, procedures, subroutines, program code segments, drivers, firmware, microcode, circuits, data, databases, data structures, tables, arrays, or variables. Components and modules or units may be combined into a smaller number of larger components and modules or units or may be divided into a larger number of smaller components and modules or units, while maintaining the same functionality.

According to an embodiment of the present disclosure, a "module" or a "unit" may be implemented with a processor and a memory. The term "processor" should be interpreted broadly to include a general-purpose processor, central processing unit (CPU), microprocessor, digital signal processor (DSP), controller, microcontroller, state machine, and/or the like. In some contexts, the "processor" may refer to an application-specific integrated circuit (ASIC), a programmable logic device (PLD), a field programmable gate array (FPGA), and/or the like. The 'processor' may refer to, for example, a combination of a DSP and a microprocessor, a combination of plural microprocessors, a combination of one or more microprocessors coupled with a DSP core, or a combination of other processing devices. In addition, the term "memory" should be interpreted broadly to include any electronic component capable of storing electronic information. The "memory" may refer to various types of processor-readable media such as random-access memory (RAM), read-only memory (ROM), non-volatile random access memory (NVRAM), programmable read-only memory (PROM), erasable-programmable read-only memory (EPROM), electrically erasable PROM (EEPROM), flash memory, magnetic or optical data storage, and/or registers. The memory is in electronic communication with a processor when the processor can read information from the memory and/or write information to the memory. The memory integrated into a processor is in electronic communication with the processor.

In the present disclosure, the term "system" may include, but not limited to, at least one of a server device and a cloud device. For example, a system may include one or more server devices. As another example, a system may include one or more cloud devices. As another example, a system may include a server device and a cloud device operating together.

In this disclosure, the sizes and relative sizes of areas illustrated in the drawings may be exaggerated to present a clearer explanation of sizing of an element. That is, sizes shown in the drawings are only for the convenience of understanding and are not limited thereto. In addition, a flowchart and its corresponding description shown in the drawings are exemplary, and the method, system, or process of a flowchart and its corresponding description may be implemented differently in some embodiments. For example, one or more steps may be omitted, the order of steps may be changed, one or more steps may be performed in an overlapping manner, or one or more steps may be repeated multiple times.

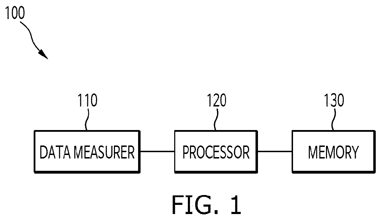

FIG. 1 is a schematic diagram of a system 100 for predicting a performance of a secondary battery according to an embodiment of the present disclosure. The system 100 for predicting the performance of the secondary battery may include a data measurer 110, a processor 120, and a memory 130.

The data measurer 110 may measure experiment data of a secondary battery required for electrochemical modeling. For example, the data measurer 110 may repeatedly charge and discharge an experimental secondary battery under various electrode conditions to measure C-rate charge data and discharge data for the various electrode conditions. As another example, the data measurer 110 may discharge an experimental secondary battery to measure open circuit voltage (OCV) of the experimental secondary battery and measure the direct current internal resistance (DCIR) data of the experimental secondary battery based on the open circuit voltage. The data measurer 110 is not limited to the above examples. In some embodiments, experiment data of a secondary battery may include C-rate charge data and discharge data for specific electrode conditions, electrochemical impedance spectroscopy (EIS) data, direct current internal resistance (DCIR) data, galvanostatic intermittent titration technique (GITT) data, derivative of voltage over capacity (dv/dq) data, particle size distribution (PSD) data, and/or the like. The data measurer 110 may monitor the secondary battery in real time to collect monitoring data, periodically, over regular intervals, or aperiodically, and transfer the monitoring data of the secondary battery to the processor 120 and/or the memory 130.

The processor 120 should be broadly construed to include a general purpose processor, a central processing unit (CPU), a microprocessor, a digital signal processor (DSP), a controller, a microcontroller, a state machine, or the like. In some environments, the processor 120 may refer to an application-specific integrated circuit (ASIC), a programmable logic device (PLD), a field programmable gate array (FPGA), or the like. The processor 120 may also refer to a combination of processing devices, such as a combination of a DSP and a microprocessor, a combination of plural microprocessors, a combination of one or more microprocessors coupled with a DSP core, or any combination thereof.

The processor 120 may be configured to process instructions of a computer program by executing basic arithmetic, logic, and input/output operations. According to some embodiments of the present disclosure, the processor 120 can receive design conditions of a secondary battery and experiment data of the secondary battery. Additionally, the processor 120 may obtain model parameters based on the experiment data and electrochemical models. In addition, the processor 120 may generate an electrochemical model library including the model parameters and may predict the electrical performance of the secondary battery according to the design conditions based on the generated electrochemical model library. The electrical performance of the secondary battery may include at least one of the charge capacity, discharge capacity, or C-rate characteristic of the secondary battery.

The memory 130 should be broadly construed to include any electronic component capable of storing electronic information. The memory 130 may refer to various types of processor-readable media, such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, or the like. If the processor 120 can read information from and/or write information to the memory 130, the memory 130 is said to be in electronic communication with the processor 120. The memory 130 integrated in the processor 120 is in electronic communication with the processor 120.

In some embodiments, the memory 130 may include a non-transitory computer-readable recording medium. In some embodiments, the memory 130 may include a permanent mass storage device. Further, a permanent mass storage device distinct from the memory may be included in the system 100 for predicting the performance of the secondary battery, or may be included in an apparatus connected by wire or wirelessly to the system 100 for predicting the performance of the secondary battery. As another example, the memory 130 may be included in the processor 120.

According to an embodiment, the memory 130 may store an operating system and at least one program code (e.g., program code for predicting the performance of the secondary battery). Additionally, the memory 130 may store experiment data on a secondary battery received from the data measurer 110. In addition, the memory 130 may store an electrochemical model library received from the processor 120.

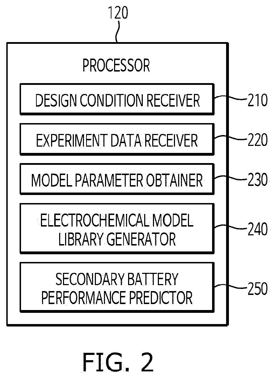

FIG. 2 is a block diagram of the internal structure of a processor 120 of the system for predicting the performance of the secondary battery according to an embodiment of the present disclosure. The processor 120 includes a design condition receiver 210, an experiment data receiver 220, a model parameter obtainer 230, an electrochemical model library generator 240, and a secondary battery performance predictor 250. The processor 120 may correspond to the processor 120 in FIG. 1.

In an embodiment, the design condition receiver 210 may receive design condition information of a secondary battery. The design condition information of the secondary battery may include at least one of electrode condition information or active material condition information. The electrode condition information of the secondary battery may include a thickness of the positive electrode plate, a thickness of the negative electrode plate, a width of the positive electrode plate, a width of the negative electrode plate, and/or the like. Additionally, the active material condition information may include an active material substance, a mixture density, a mass per unit area of the mixture (loading level), and/or the like. The design condition information of the secondary battery may be information input by the user or information received by the design condition receiver 210 from an external device. The design condition receiver 210 may forward the received design condition information of the secondary battery to the secondary battery performance predictor 250 or the memory.

The experiment data receiver 220 may receive experiment data of the secondary battery from the data measurer (e.g., data measurer 110 in FIG. 1). In an embodiment, experiment data of the secondary battery may include the C-rate charge data and the discharge data for specific electrode conditions, the electrochemical impedance spectroscopy (EIS) data, the direct current internal resistance (DCIR) data, the galvanostatic intermittent titration technique (GITT) data, the derivative of voltage over capacity (dv/dq) data, the particle size distribution (PSD) data, and/or the like. The experiment data receiver 220 may receive experiment data of the secondary battery from the data measurer 110 in real time, periodically at predetermined intervals, or aperiodically. The experiment data receiver 220 may forward the received experiment data of the secondary battery to the model parameter obtainer 230 or the memory.

The model parameter obtainer 230 can obtain model parameters based on the experiment data and the electrochemical model of the secondary battery received from the experiment data receiver 220. The model parameters include a first model parameter associated with electrolyte properties, a second model parameter associated with active material properties, and a third model parameter associated with electrode plate properties. The model parameter obtainer 230 may transfer the model parameters to the electrochemical model library generator 240 and/or to the memory.

The model parameter obtainer 230 may obtain first model parameters associated with electrolyte properties from first experiment data through an Advanced Electrolyte Model (AEM) simulation. The AEM simulation may operate as a model that analyzes and optimizes electrolyte properties in an electrochemical system.

The model parameter obtainer 230 may obtain second model parameters associated with active material properties from second experiment data through a discrete element method (DEM) simulation. The DEM simulation is a numerical technique for simulating behavior of particulate materials and may operate as a simulation technique for analyzing an interaction among active material fine particles included in the second experiment data.

The model parameter obtainer 230 may obtain third model parameters associated with electrode plate properties from third experiment data through a Newman model. The Newman model is an electrochemical model for interpreting the characteristics of a secondary battery electrode plate. The Newman model is a simulation model for predicting the charge and discharge processes of a secondary battery based on ion movement, electrical potential change, and/or electrolyte diffusion in a porous electrode structure.

The model parameter obtainer 230 may apply experiment data of a secondary battery to an electrochemical model to generate prediction data and may optimize the model parameters by comparing the experiment data with the prediction data. For example, the model parameter obtainer 230 may optimize the model parameters by (i) comparing the experiment data and the prediction data to calculate an error value and (ii) determining whether the calculated error value is greater than or equal to a predetermined threshold value.

The model parameter obtainer 230 may generate input data for an electrochemical model based on the experiment data and may apply the input data to the electrochemical model to obtain the prediction data. In addition, it may determine whether the error value between the experiment data and the prediction data is greater than or equal to the predetermined threshold value. Specifically, the model parameter obtainer 230 may compare, at equal intervals, profiles of the experiment data and profiles of the prediction data. The model parameter obtainer 230 may compare, at equal intervals, the profiles of the experiment data and the profiles of prediction data to calculate an average value of relative error values.

Upon determining that the error value between the experiment data and the prediction data is greater than or equal to the predetermined threshold value, the model parameter obtainer 230 may generate new input data through an optimization algorithm. The optimization algorithm may include at least one of a particle swarm optimization (PSO) algorithm, a genetic algorithm (GA), or a Bayesian algorithm. The model parameter obtainer 230 may optimize the model parameters through the above-described process including comparing the experiment data with the prediction data.

The electrochemical model library generator 240 may generate an electrochemical model library including optimized model parameters received from the model parameter obtainer 230. The electrochemical model library may include model parameters optimized according to various design conditions of a secondary battery. For example, the electrochemical model library may include an electrolyte library including first model parameters associated with electrolyte properties, an active material library including second model parameters associated with active material properties, and an electrode plate library including third model parameters associated with electrode plate properties. The electrochemical model library generator 240 may periodically or aperiodically transfer information of the electrochemical model library to the secondary battery performance predictor 250 or the memory as the electrochemical model library is updated in real time.

The secondary battery performance predictor 250 may predict the electrical performance of the secondary battery according to the design conditions received from the design condition receiver 210 and information of the electrochemical model library received from the electrochemical model library generator 240. The electrical performance of a secondary battery may include at least one of the charge capacity, the discharge capacity, or the C-rate characteristic of the secondary battery.

The secondary battery performance predictor 250 may predict the electrical performance of a secondary battery according to design conditions of a mixed material. The mixed material includes a mixture of multiple ingredients having different properties. In some embodiments, active material conditions may include secondary battery performance prediction conditions of a mixed material composed of lithium iron phosphate (LFP) and lithium manganese iron phosphate (LMFP) materials.

In FIG. 2, the individual components of the processor 120 represent distinct functional elements, and multiple components may be integrated with one another in a physical environment. Alternatively, the components of the processor 120 may be separate from each other in a physical environment. Additionally, although illustrated as a single processor in FIG. 2, the processor 120 may be a multi-core processor including multiple processors (or cores).

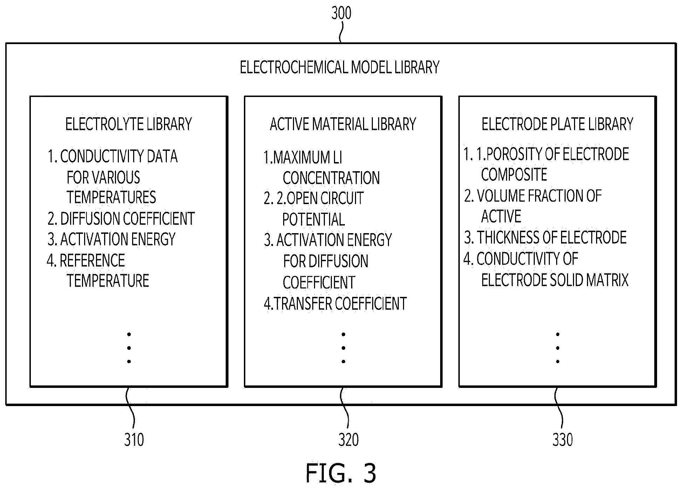

FIG. 3 is a diagram of an electrochemical model library 300 of the system for predicting the performance of the secondary battery according to an embodiment of the present disclosure. The electrochemical model library 300 may include an electrolyte library 310 storing electrolyte properties, an active material library 320 storing active material properties, and an electrode plate library 330 storing electrode plate properties.

The electrochemical model library 300 may include model parameters optimized according to various secondary battery design conditions. Each model parameter stored in the electrochemical model library 300 may be determined through an optimization process of comparing experiment data of a secondary battery with prediction data derived through an electrochemical model. In addition, the above optimization process may be performed in real time by the processor 120 (FIG. 1). Hence, the processor 120 may update the electrochemical model library 300 with an optimized model parameter. When the processor 120 makes an update with the optimized model parameter, an existing parameter predicted under the same conditions may be excluded from the electrochemical model library 300. Therefore, as the processor 120 repeats the process of updating the electrochemical model library 300, model parameter values having improved accuracy may be stored in the electrochemical model library 300.

The electrolyte library 310 may include a plurality of model parameters associated with electrical conductivity of an electrolyte at various electrolyte temperatures, electrical conductivity of the electrolyte at various electrolyte concentrations, a diffusion coefficient related to a specific electrolyte, activation energy related to a specific electrolyte, and the like. But the model parameters included in the electrolyte library 310 are not limited thereto. The model parameters included in the electrolyte library 310 may be used to predict the electrical performance of a secondary battery based on design conditions.

In some embodiments, the active material library 320 may include a plurality of model parameters associated with a specific active material, such as a maximum lithium-ion concentration, an open circuit potential, activation energy for a diffusion coefficient, or a transfer coefficient. In addition, the active material library 320 may include not only characteristic data of the active material in a single state but also characteristic data of the active material in a mixed state. For example, the characteristic data for the active material in a single state may include a distribution and a size of active material particles, and the characteristic data for the active material in a mixed state may include a density, a porosity, or the like according to a mixing ratio of the active material; however, the model parameters included in the active material library 320 are not limited thereto. The model parameters included in the active material library 320 may be used to predict the electrical performance of a secondary battery according to design conditions.

In some embodiments, the electrode plate library 330 may include a plurality of model parameters associated with a porosity of the electrode composite, a volumetric ratio of the electrode plate to the mixture, a thickness of the electrode plate, a width of the electrode plate, an electrical conductivity of the solid matrix of the electrode plate, and/or the like. The electrode plate library 330 may include model parameters for a positive electrode and a negative electrode; however, the model parameters included in the electrode plate library 330 are not limited thereto. The model parameters included in the electrode plate library 330 may predict the electrical performance of a secondary battery according to design conditions.

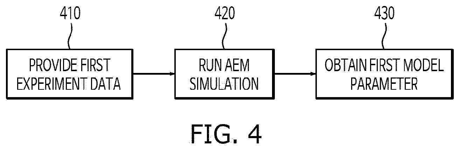

FIG. 4 is a diagram of a method for obtaining a first model parameter 430 according to an embodiment of the present disclosure. The first experiment data 410 related to electrolyte properties may be applied to the advanced electrolyte model (AEM) simulation 420 to obtain the first model parameter 430. The first experiment data 410 related to electrolyte properties may include data according to electrolyte composition conditions. For example, the first experiment data 410 associated with electrolyte properties may include data related to electrical conductivity of an electrolyte with a composition condition in which EC (ethylene carbonate), EMC (ethyl methyl carbonate), and DMC (dimethyl carbonate) are mixed to achieve a ratio of 1:2:2 respectively. In addition, the AEM simulation 420 may be a model for analyzing and optimizing electrolyte properties in an electrochemical system. The obtained first model parameter 430 may be stored in the electrolyte library 310 (FIG. 3) after undergoing an optimization process.

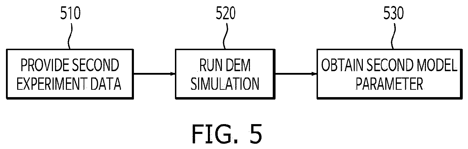

FIG. 5 is a diagram of a method of obtaining a second model parameter 530 according to an embodiment of the present disclosure. The second experiment data 510 related to active material characteristics may be applied to a discrete element method (DEM) simulation 520 to obtain the second model parameter 530. The second experiment data 510 related to active material properties may include an amount of active material, a distribution and size of active material particles, and/or the like. Additionally, the second experiment data 510 related to active material properties may include characteristic data of the active material in a mixed state. Further, the DEM simulation 520 may be used to analyze an interaction among active material fine particles included in the second experiment data related to active material properties. The second model parameter 530, once obtained, may be stored in the active material library 320 (FIG. 3) after undergoing an optimization process.

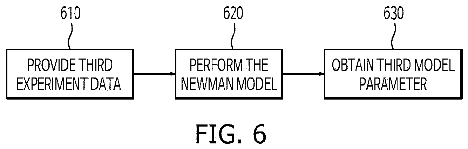

FIG. 6 is a diagram of a method of obtaining a third model parameter 630 according to an embodiment of the present disclosure. The third experiment data 610 related to electrode plate properties may be applied to the Newman model 620 to obtain the third model parameter 630. The third experiment data 610 related to electrode plate properties may include experiment data associated with an electrode plate thickness, a cell area, a substrate thickness, a substrate weight, and/or the like. The Newman model 620 may be used to predict the charge and discharge process of a secondary battery based on ion movement, an electrical potential change, an electrolyte diffusion, and/or the like in a porous electrode structure. The third model parameter 630, once obtained, may be stored in the electrode plate library 330 (FIG. 3) after undergoing an optimization process.

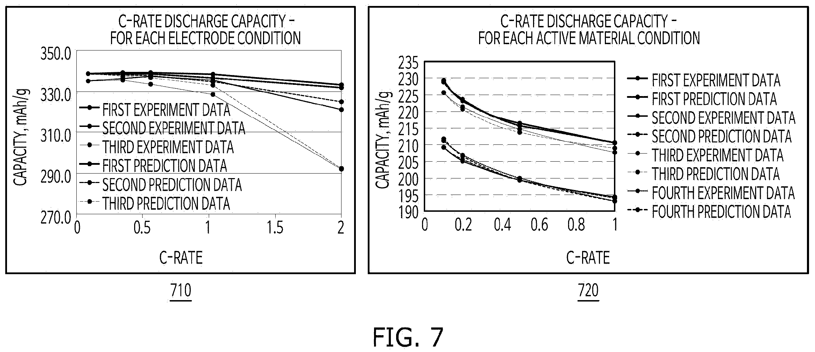

FIG. 7 shows graphs of data for predicting the performance of the secondary battery according to an embodiment of the present disclosure. A first graph 710 is a graph showing experiment data and prediction data for the C-rate discharge capacity under a specific electrode condition, and a second graph 720 is a graph showing experiment data and prediction data for the C-rate discharge capacity under a specific active material condition.

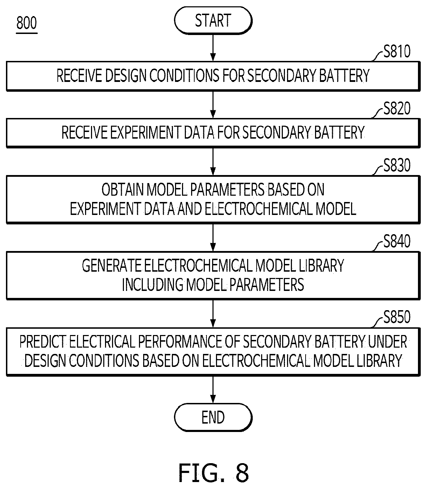

The horizontal axis of the first graph 710 indicates the C-rate, and the vertical axis of the first graph 710 indicates the discharge capacity. In the first graph 710, first to third experiment data values for specific electrode conditions and first to third prediction data values for specific electrode conditions are shown. The first experiment data is data measured experimentally for a discharge capacity of the electrode plate corresponding to 46 µm, and the first prediction data is data for predicting the discharge capacity of the electrode plate corresponding to 46 µm by using a method of predicting a performance of a secondary battery according to an embodiment of the present disclosure (see FIG. 8). Additionally, the second experiment data is data measured experimentally for the discharge capacity of the electrode plate corresponding to 55 µm, and the second prediction data is data for predicting the discharge capacity of the electrode plate corresponding to 55 µm by using a method of predicting a performance of a secondary battery according to an embodiment of the present disclosure (see FIG. 8). Additionally, the third experiment data is data measured experimentally for the discharge capacity of the electrode plate corresponding to 58 µm, and the second prediction data is data for predicting the discharge capacity of the electrode plate corresponding to 58 µm by using a method of predicting a performance of a secondary battery according to an embodiment of the present disclosure (see FIG. 8). As shown by the first graph 710, it can be seen that the data predicted through a method of predicting a performance of a secondary battery according to an embodiment of the present disclosure (see FIG. 8) exhibits a high level of accuracy when compared with data measured through actual experiments. Therefore, by predicting the electrical performance of a secondary battery under various electrode conditions and managing predictions of the electrical performance of the secondary battery as a library, the electrical performance of the secondary battery may be derived without conducting new experiments even if the design conditions change.

The horizontal axis of the second graph 720 indicates the C-rate, and the vertical axis of the second graph 720 indicates the discharge capacity. In the second graph 720, first to fourth experiment data values for specific active material conditions and first to fourth prediction data values for specific active material conditions are shown. The first experiment data is data measured experimentally for the discharge capacity of an NCA positive electrode active material, and the first prediction data is data for predicting the discharge capacity of the NCA positive electrode active material by using a method of predicting a performance of a secondary battery according to an embodiment of the present disclosure (see FIG. 8). Additionally, the second experiment data is data measured experimentally for the discharge capacity of an NCM positive electrode active material, and the second prediction data is data for predicting the discharge capacity of the NCM positive electrode active material by using a method of predicting a performance of a secondary battery according to an embodiment of the present disclosure (see FIG. 8). Additionally, the third experiment data is data measured experimentally for the discharge capacity of an Ni positive electrode active material, and the third prediction data is data for predicting the discharge capacity of the Ni positive electrode active material by using a method of predicting a performance of a secondary battery according to an embodiment of the present disclosure (see FIG. 8). In addition, the fourth experiment data is data measured experimentally for the discharge capacity of an NMX positive electrode active material, and the fourth prediction data is data for predicting the discharge capacity of the NMX positive electrode active material by using a method of predicting a performance of a secondary battery according to an embodiment of the present disclosure (see FIG. 8). As shown by the second graph 720, it can be seen that the data predicted through the method of predicting the performance of the secondary battery according to an embodiment of the present disclosure (see FIG. 8) exhibits a high level of accuracy when compared to the data measured through actual experiments. Therefore, by predicting the electrical performance of a secondary battery under various active material conditions and managing predictions of the electrical performance of the second battery as a library, the electrical performance of the secondary battery may be derived without conducting new experiments even if the design conditions change.

FIG. 8 is a method 800 of predicting the performance of the secondary battery according to an embodiment of the present disclosure. The method 800 of predicting the performance of the secondary battery may be executed by at least one processor. First, the method 800 of predicting the performance of the secondary battery may be initiated by the processor in response to receiving design conditions of a secondary battery S810. In an embodiment, the design conditions of the secondary battery may include at least one of electrode conditions or active material conditions. The electrode conditions of the secondary battery may include the thickness of the positive electrode plate, the thickness of the negative electrode plate, the width of the positive electrode plate, the width of the negative electrode plate, and/or the like. Additionally, the active material conditions may include the active material substance, the mixture density, the mass per unit area of the mixture (loading level), and/or the like.

Thereafter, the processor may receive experiment data of the secondary battery S820. The experiment data of the secondary battery may include the C-rate charge data and the discharge data for specific electrode conditions, the electrochemical impedance spectroscopy (EIS) data, the direct current internal resistance (DCIR) data, the galvanostatic intermittent titration technique (GITT) data, the derivative of voltage over capacity (dv/dq) data, the particle size distribution (PSD) data, and/or the like.

Thereafter, the processor may obtain model parameters based on the experiment data and the electrochemical model S830. In an embodiment, the model parameters may include a first model parameter associated with electrolyte properties, a second model parameter associated with active material properties, and a third model parameter associated with electrode plate properties.

In an embodiment, the processor may obtain the first model parameters associated with electrolyte properties from first experiment data through an Advanced Electrolyte Model (AEM) simulation. The AEM simulation may be a model that analyzes and optimizes electrolyte properties in an electrochemical system.

In an embodiment, the processor may obtain the second model parameters associated with active material properties through a discrete element method (DEM) simulation. The DEM simulation may be used to analyze the interaction among active material fine particles included in the second experiment data associated with active material properties.

In an embodiment, the processor may obtain third model parameters associated with electrode plate properties through a Newman model. The Newman model may be used to predict the charge and discharge process of a secondary battery based on ion movement, the electrical potential change, and/or the electrolyte diffusion in a porous electrode structure.

Thereafter, the processor may generate an electrochemical model library including the model parameters S840. In addition, the processor may predict the electrical performance of the secondary battery under the design conditions of the electrochemical model library S850.

In some embodiments, the active material conditions among the design conditions may include a condition for a mixed material in which multiple ingredients having different properties are mixed. For example, the active material conditions may include secondary battery performance prediction conditions for a mixed material composed of lithium iron phosphate (LFP) and lithium manganese iron phosphate (LMFP) materials.

In an embodiment, the predicted electrical performance of the secondary battery may include at least one of the charge capacity, the discharge capacity, or the C-rate characteristic of the secondary battery.

The flowchart of FIG. 8 and the description above are exemplary embodiments of the present disclosure. But the scope of the present disclosure is not limited to the method set forth in the flowchart of FIG. 8 and the descriptions above. For example, one or more steps in the flowchart and/or the description thereof may be added, changed, and/or deleted, the order of one or more steps may be changed, and one or more steps may be executed simultaneously.

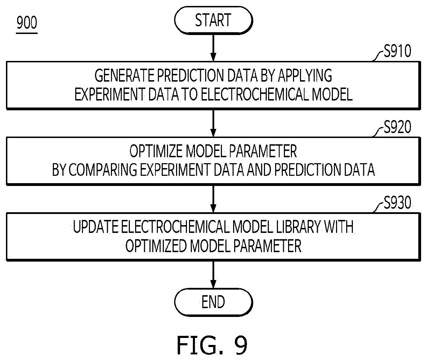

FIG. 9 is a flowchart for explaining a process 900 for updating the electrochemical model library according to an embodiment of the present disclosure. The process 900 of updating the electrochemical model library may be initiated by a processor, which generates prediction data by applying experiment data to the electrochemical model S910. And the processor may optimize a model parameter by comparing the experiment data and the prediction data S920. Particularly, the processor may optimize the model parameter by comparing the experiment data with the prediction data to calculate an error value and by determining whether the calculated error value is greater than or equal to a predetermined threshold value.

Thereafter, the processor may update the electrochemical model library with the optimized model parameter S930. When the processor updates the electrochemical model library with the optimized model parameter, an existing parameter predicted under the same conditions may be excluded from the electrochemical model library. Therefore, as the processor repeats the process of updating the electrochemical model library, model parameter values with improved accuracy may be stored in the electrochemical model library.

The method set forth in the flowchart of FIG. 9 and the descriptions above are exemplary embodiments of the present disclosure. But the present disclosure is not limited to method in the flowchart of FIG. 9 and the description thereof. For example, one or more steps in the flowchart and/or the description thereof may be added, changed, and/or deleted, the order of one or more steps may be changed, and one or more steps may be executed simultaneously.

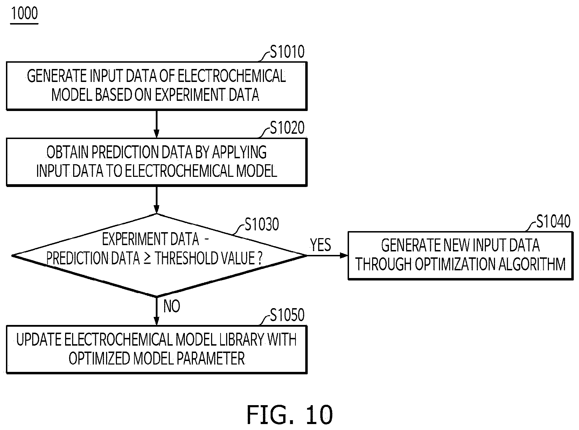

FIG. 10 is a flowchart for explaining a process 1000 of optimizing model parameters according to an embodiment of the present disclosure. The process 1000 of optimizing model parameters may be initiated by the processor that generates input data of the electrochemical model based on experiment data of the secondary battery S1010. The experiment data of the secondary battery may include the C-rate charge data and discharge data for specific electrode conditions, the electrochemical impedance spectroscopy (EIS) data, the direct current internal resistance (DCIR) data, the galvanostatic intermittent titration technique (GITT) data, the derivative of voltage over capacity (dv/dq) data, the particle size distribution (PSD) data, and/or the like. The input data may include (i) data converted from the experiment data so that it may be applied to the electrochemical model and (ii) data on a parameter to be identified through an electrochemical model simulation. The data on a parameter to be identified through an electrochemical model simulation may include a charge transfer resistance (R_{ct}), an ohmic resistance, an ionic resistance, transfer coefficients of the positive electrode and the negative electrode, a diffusion coefficient, an electrical conductivity of the material, and/or the like.

Thereafter, the processor may obtain the prediction data by applying the input data to the electrochemical model S1020. The processor may determine whether the error value between the experiment data and the prediction data is greater than or equal to a predetermined threshold value S1030. Specifically, the processor may compare profiles of the experiment data and the prediction data at equal intervals. Particularly, the processor may compare profiles of the experiment data and the prediction data at equal intervals to calculate an average value of relative error values. The relative error value may be defined as a ratio of the difference between the experiment data and the prediction data to the experiment data. The predetermined threshold value may be 5 percent, but the present disclosure is not limited thereto.

Thereafter, upon determining that the error value between the experiment data and the prediction data is greater than or equal to the predetermined threshold value (indicated by "Yes" in FIG. 10), the processor may generate new input data through an optimization algorithm S 1040. The optimization algorithm may include at least one of a particle swarm optimization (PSO) algorithm, a genetic algorithm (GA), or a Bayesian algorithm. On the other hand, upon determining that the error value between the experiment data and the prediction data is less than the predetermined threshold value (indicated by "No" in FIG. 10), the processor may update the electrochemical model library with the optimized model parameter S1050. In an embodiment, the model parameter optimization process described in the present disclosure may be performed for a predetermined number of iterations to obtain an optimized model parameter. If an optimized model parameter with an error value less than a predetermined threshold is not obtained within the predetermined number of iterations, the model parameter optimization process may be terminated.

The method in the flowchart of FIG. 10 and the description aboves are exemplary embodiments of the present disclosure. But the scope of the present disclosure is not limited to the flowchart of FIG. 10 and the descriptions thereof. For example, one or more steps in the flowchart and/or the description thereof may be added, changed, and/or deleted, the order of one or more steps may be changed, and one or more steps may be executed simultaneously.

Preferred embodiments of the present disclosure have been disclosed for the purpose of illustration, and those skilled in the art with knowledge of the present disclosure will be able to make various modifications, changes, and additions in accordance with the scope of the present disclosure.

Those skilled in the art to which the present disclosure pertains will appreciate that various substitutions, modifications, and changes can be made without departing from the technical scope of the present disclosure. Thus, the present disclosure is not limited to the above-described embodiments and the attached drawings.

Although the present disclosure has been described above with respect to embodiments thereof, the present disclosure is not limited thereto. Various modifications and variations can be made thereto by those skilled in the art within the scope of the present disclosure.

### [DESCRIPTION OF SOME REFERENCE SYMBOLS]

100: system for predicting a performance of a secondary battery
110: data measurer
120: processor
130: memory

## Claims

1. A method (800) of predicting an electrical performance of a secondary battery, the method comprising:
receiving (S810) design conditions of the secondary battery, the design conditions of the secondary battery including at least one of an electrode condition or an active material condition;
receiving (S820) experiment data of the secondary battery;
obtaining (S830) model parameters based on the experiment data and an electrochemical model;
generating (S840) an electrochemical model library (300) that includes the model parameters; and
predicting (S850) the electrical performance of the secondary battery, having the design conditions based on the electrochemical model library (300).

2. The method (800) of claim 1, wherein the electrical performance of the secondary battery comprises at least one of a charge capacity, a discharge capacity, or a C-rate characteristic of the secondary battery.

3. The method (800) of claim 1 or 2, further comprising:
generating (S910) prediction data by applying the experiment data to the electrochemical model;
optimizing (S920) the model parameters by comparing the experiment data and the prediction data; and
updating (S930) the electrochemical model library (300) with the optimized model parameters.

4. The method (800) of claim 3, further comprising:
generating (S1010) input data of the electrochemical model based on the experiment data;
obtaining (S1020) the prediction data by applying the input data to the electrochemical model; and
generating (S1040) new input data through an optimization algorithm in response to determining that an error value between the experiment data and the prediction data is greater than or equal to a predetermined threshold value.

5. The method (800) of claim 4, wherein generating (S1040) new input data through the optimization algorithm includes generating new input data using at least one of a particle swarm optimization algorithm, a genetic algorithm, or a Bayesian algorithm.

6. The method (800) of any one of the preceding claims, wherein obtaining (S830) the model parameters includes obtaining a first model parameter (430) associated with electrolyte properties, obtaining a second model parameter (530) associated with active material properties, and obtaining a third model parameter (630) associated with electrode plate properties.

7. The method (800) of any one of the preceding claims, wherein obtaining (S830) the model parameters includes obtaining a first model parameter (430) related to electrolyte properties from first experiment data (410) through an advanced electrolyte model simulation (420).

8. The method (800) of any one of the preceding claims, wherein obtaining (S830) the model parameters comprises obtaining a second model parameter (530) related to active material properties from second experiment data (510) through a discrete element method simulation (520).

9. The method (800) of any one of the preceding claims, wherein obtaining (S830) the model parameters comprises obtaining a third model parameter (630) related to electrode plate properties from third experiment data (610) through a Newman model (620).

10. The method (800) of any one of the preceding claims, wherein receiving (S810) design conditions of the second battery includes receiving the active material condition, the active material condition including a condition for a mixed material in which multiple ingredients having different properties are mixed.

11. A system (100) for predicting an electrical performance of a secondary battery, the system (100) comprising:
a memory (130); and
at least one processor (120) connected to the memory (130) and configured to execute at least one computer-readable program stored in the memory (130) to thereby cause the at least one processor (120) to be configured to:
receive design conditions of the secondary battery, the design conditions including at least one of an electrode condition or an active material condition;
receive experiment data of the secondary battery;
obtain model parameters based on the experiment data and an electrochemical model;
generate an electrochemical model library (300) comprising the model parameters; and
predict the electrical performance of the secondary battery, having the design conditions, based on the electrochemical model library (300).

12. The system (100) of claim 11, wherein the electrical performance of the secondary battery comprises at least one of a charge capacity, a discharge capacity, or a C-rate characteristic of the secondary battery.

13. The system (100) of claim 11 or 12, wherein the at least one processor (120) is further configured to:
generate prediction data by applying the experiment data to the electrochemical model;
optimize the model parameters by comparing the experiment data and the prediction data; and
update the electrochemical model library (300) with the optimized model parameters.

14. A computer program comprising instructions which, when the program is executed by a computer, cause the computer to carry out the method of any one of the claims 1 to 10

15. A computer-readable medium comprising instructions which, when executed by a computer, cause the computer to carry out the method of any one of the claims 1 to 10.
